# EUROPEAN PATENT APPLICATION

(11) **EP 1 215 613 A1**
(43) Date of publication of application: **19.06.2002**
(21) Application number: 00127559.3
(22) Date of filing: 15.12.2000
(51) Int. Cl.: G06F 19/00

(54) **A digital molecular integrator**

(71) Applicant: La Clair, James J., Dr., 10435 Berlin (DE)
(72) Inventor: La Clair, James J., Dr., 10435 Berlin (DE)
(74) Representative: Knauthe Paul Schmitt

(57) **Abstract**

The invention presents a method and a digital electronic device to create, read, write and communicate molecular information. An apparatus that identifies molecular species by conducting a digitally-encoded microelectronic script (1) through a integrated circuit that contains a read channel (2), a write channel (3) and molecular input/output device (4) containing a molecular motor (5), and portal (6). The device uses a digital script (1) to organize, create and read an array of specific molecular queries. Its system can be networked to provide microelectronic affinity matrixes that directly communicate with instruments of telecommunication and computing.

## Description

The present invention relates to molecular sensing methods and systems. The invention presents a method and a digital electronic device to create, read, write and communicate molecular information.

Molecular screening has been profoundly advanced through the development of affinity matrixes. A series of techniques developed on cells, beads, or chips now identify a specific molecule based on its interaction with receptor in an optical field. These techniques have been accompanied by the development of bioinformatic information systems, such as that seen in U.S. Pat. No. 6,136,541. The union between personal computer, software, and optical reader allows users to screen antibodies, DNA, RNA, proteins, carbohydrates, natural products, pharmaceuticals or other small molecules. In these systems, a computer network and software serve as a creator and/or processor and the optical reader as a molecular input output (mI/O) device. The complexity of the information transfer between optical signal and digital code has created a need for extensive bioinformatic systems.

Recent attention has turned toward developing mI/Os based on sensing elements that correlate molecular specificity with a microelectrochemical response. This advance simplifies requirements of bioinformatic software, by directly placing the mI/O in circuit with a conventional microprocessor. A series of patents including U.S. Pat. 6,051,380 describe a system for gene analysis that uses CMOS circuitry to organize oligonuclotide probes for DNA assays. Other disclosures directly use molecular recognition to regulate integrated circuitry. U.S. Pat No. 6,117,642 describes a biosensor that uses a light diode to connect bioluminescence with a CMOS microchip. Other inventions such as that described in U.S. Pat No. 6,048,692 6,060,023, 6,060,317, 6,060,242, 6,016,686, 6,130,037, 6,132,971, 6,133,046, or 6,110,339, are based on monitoring electrical transmission between molecularly encoded electrodes. Other disclosures describe the combination of a molecular sensor with an integrated circuit, such as that described in 6,122,704, 6,117,630, 6,096,172. All of these discoveries focus on adapting molecular binding to alter the function of conventional microelectronic systems.

Other efforts have focused on the development of molecular devices. These efforts now provide the molecular equivalent of wires as described in U.S. Pat No. 6,146,227, 6,140,503, 6,107,038, or 6,096, 273 switches as described in U.S. Pat No. 6,128,214, 6,130,096, 6,121,495, 5,905,679, 6,083,726, or, 5,659,418, transistors as described in U.S.

Pat No. 5,589,692. To date, these efforts have focused on replicating conventional microelectronic elements with molecular analogs.

Other efforts have described the use of optical interferometry to sense molecular events as given by U.S. Pat No. 6,033,913, 6,016,686, or 6,111,416. Few inventions describe the use of electronic interferometry to diagnose a molecular event. Of the few that exist, such as U.S. Pat. 5,928,181 or 6,138,541, no invention to date has described the application of molecular motors to electronic interferometry. Furthermore, no invention, process or method given to date describes an integrated circuit that examines a molecular analyses using digital microelectronic data.

It is therefore an object of the present invention to provide a method and an apparatus for reading, writing and communicating information on molecular species using digital microelectronic data in order to facilitate, e.g., the diagnostic methods known in the art. This object is achieved by the method according to claim 1 and the apparatus according to claim 10.

The complete disclosure of the preceding member of this series, EU 00120417.1, is herein incorporated by reference. EU 00120417.1 describes the operating system of DITOM. Especially incorporated herein is the system disclosed in EU 00120417.1, which is defined as any physical or mathematical element, gathered information about a molecular species by examining how a molecule or group of molecules directly alters a flux of digital data. The operating system according to EU 00120417.1 inputs molecules and outputs their identity, reactivity and/or expression directly in the form of error in digital code. The principles of this method and applications extend a direct conduit between molecular and digital information, and generate a primary system to communicate and network molecular intelligence. This invention describes a new and inventive mI/O device based on a molecular motor regulator. The system determines the identity and/or expression of a molecule using a microelectronic form of DITOM.

The method for reading, writing and communicating information on molecular species according to the present invention comprises generating or transiting digital data 1 by or via a user-accessible portal 6, transiting digital data 1 via at least one read channel 2 to at least one microelectronic molecular input/output device 4, transiting the digital data 1 through the at least one molecular input/output device 4, transiting the digital data via at least one write channel 3 to portal 6.

The apparatus for reading, writing and communicating information on molecular species according to the present invention comprises an integrated circuit containing a user-accessible portal 6; means for generating or transiting digital data 1 by or via the portal 6; at least one read channel 2; means for transiting the digital data 1 through the read channel 2; at least one microelectronic molecular input/output device 4; means for transiting the digital data 1 from the read channel 2 to the at least one microelectronic molecular input/output device 4; at least one write channel 3; means for transiting the digital data 1 from the molecular input/output device 4 via the write channel 3 to the portal 6.

Further improvements and preferred embodiments are subject of the dependent claims.

### BRIEF DESCRIPTION OF THE DRAWINGS

- FIG. 1: depicts circuitry of the molecular integrator;
- FIG. 2: outlines the application of DITOM to this invention;
- FIG. 3: provides a preferred embodiment of the molecular input/output device;
- FIG. 4: provides the mathematical formulation used to process the digital data obtained when reading the mI/O;
- FIG. 5: provides a preferred embodiment of a spring-operated molecular motor;
- FIG. 6: provides a preferred embodiment of a dynamic-control molecular motor;
- FIG. 7: provides a preferred embodiment of a rotary molecular motor;
- FIG. 8: provides a preferred embodiment of a switchable molecular motor;
- FIG. 9: provides a preferred embodiment of the microelectronic script;
- FIG. 10: outlines the process of writing and reading with the integrator;
- FIG. 11: provides a preferred embodiment for an affinity matrix based on networks of integrators;
- FIG. 12: provides an example of spring-operated molecular motor;
- FIG. 13: provides an example of a rotary molecular motor
- FIG. 14: provides an example of switchable molecular motor
- FIG. 15: provides suggested examples for the interferon

The figures illustrate by way of non limiting examples preferred embodiments of the invention.

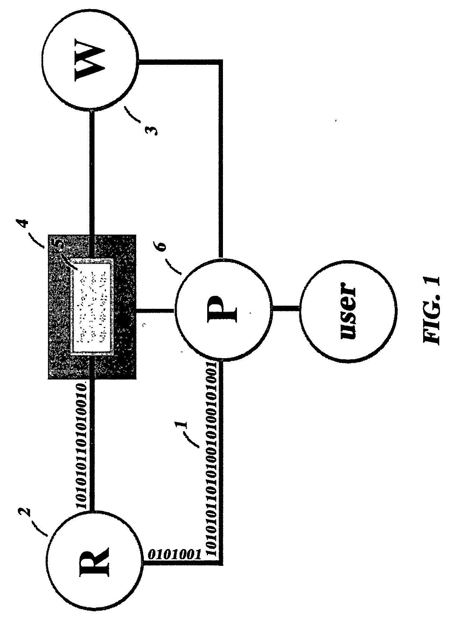

FIG. 1 illustrates the molecular integrator device. This integrator contains digital data in form of a digital microelectronic script 1, a read channel 2, a write channel 3, a molecular input/output (mI/O) device 4 with a molecular motor 5 and a portal 6. The user contacts the integrator through portal 6 either directly through an entry device, including but not limited to a keyboard, mouse or number pad, and/or remotely through a communication unit, including but not limited to a telephone, modem, infrared file exchange, pulsed-laser and/or cellular phone. The portal communicates using digital data in form of a script 1. Script 1 is designed such that it represents each molecule with unique digital identity. This script also instructs mI/O 4 to either print or read.

Portal 6 builds script 1 by generating or transiting pulses of electronic current preferably at a rate that exceeds 0.1 GHz. These pulses can be expressed in waveforms, wherein box wave character is preferred. When the pulse reaches its maximum, a digit of 1 is given. Similarly, as the current reaches its minimum, a digit of 0 is given. Series of digital pulses are then grouped into strings that digitally represents the microelectronic script 1. During writing, script 1 is directed through write channel 2 directly to a printing mechanism in mI/O 4. For reading, script 1 is sent through read channel 2 to mI/O 4 where it is applied to molecular motor 5. Information management in this motor 5 is operated using the DITOM system.

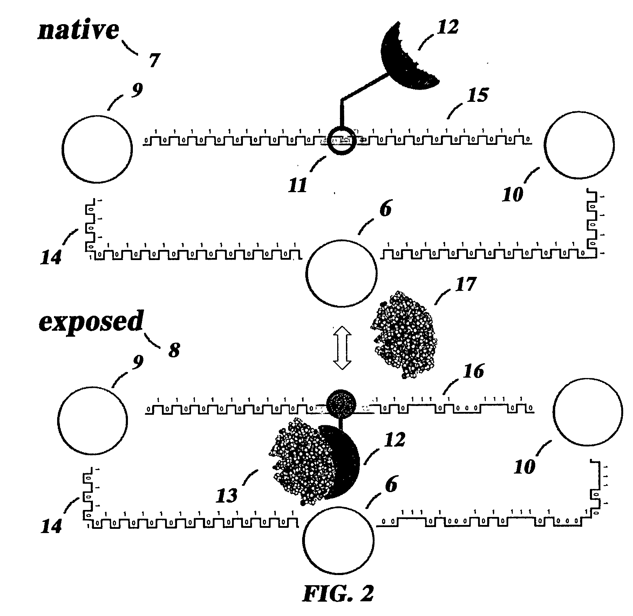

FIG. 2 illustrates this application. During reading, script 1 is passed from portal 6 through read channel 2 to creator 9. This script is called M(x,y) 14 as it reaches creator 9. In native state 7, creator 9 transmits M(x,y) 14 through motor 11 to processor 10. Motor 11 is a molecular species that exist in a state of dynamic equilibrium. The motor screens for molecule 17 by monitoring the alteration of this equilibrium upon converting mI/O 4 to an exposed state 8. An outline of this process follows.

In all states, the dynamics of motor 5 regulates the transmission of 14 to processor 10. In the native state 7, N (x, y) 15 represent the data received at processor 10. This data set 15 is a algebraic or statistical function of 14. The Motor 11 is then exposed to at least one molecule 17. The at least one molecule 17 binds to receptor 12. This binding creates a new or exposed state 8. In exposed state 8, the dynamic equilibrium in the motor 13 is different than that in native state motor 11. This change alters the transmission of M(x,y) 14, as given the receipt a new data set E(x,y) 16 at the processor 10. The identity of the molecule 17 is decoded by its binding to receptor 12 by monitoring the difference between E(x,y) 16 and N(x,y) 15 with regards to the structure of M(x,y) 14.

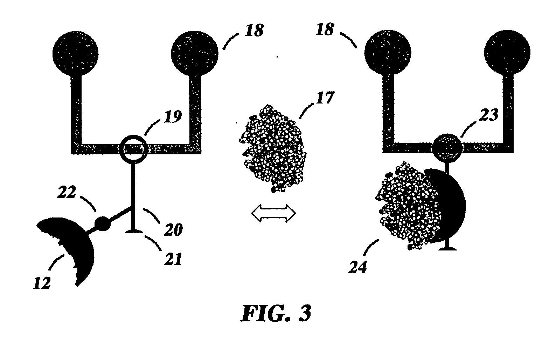

The operation of the molecular motor is outlined in FIG. 3. This motor is built using synthetic chemistry such that it contains interferon 19 or 23, template 20, mount 21, linker 22, and receptor 12. The interferon 19 or 23 is a unit that plugs the motor into electrical circuit 18. This circuit is integrated through the read channel 2 and mI/O 4 as shown in FIG 1. Template 20 is a molecular moiety that serves as the foundation and housing of the motor. Mount 21 is a molecular moiety that can fix the motor to a surface. Preferably, mount 21 contains functionality that allows it to be attached to polycarbonate, glass, or gold. Linker 22 provides a handle to affix the receptor 12 during writing stage. Preferably linker 22 contains a functionality that attaches to at least one molecule of a oligonucleotide, a protein, a carbohydrate, a carboxylic acid or ester.

In native state 7, receptor 12 is free of molecule 17. In this state, interferon 19 alters the passing of M(x,y) 14 through circuit 18 with defined distortion, transmitting N(x,y) 16. As molecule 17 binds to receptor 12, as shown in 24, interferon 19 is changed to an exposed position 23. The exposed interferon 23 can be viewed as electrochemically activated. The interaction between 23 and 18 distorts transmission of script 1 creating error as given in E(x,y) 16. This error is expressed altering structure and/or threshold intensity of pulses in 14. This alteration appears to processor 10 as error. The motor integrates a molecular species by transiting receptor-molecule binding over an electrointerferometric distortion. The process operates through the interferon and is defined by the conversion of 19 in native state 7 to 23 in exposed state 8, wherein state 7 has a motor 11 and interferon 19, and state 8 has motor 13 and interferon 23. Therefore, the interferon 19; 23 changes the electrical properties of a microelectronic channel upon binding or recognizing or reacting of at least one molecule to the molecular motor's receptor 12.

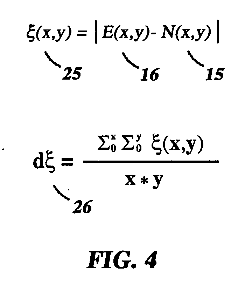

Signal processing of this system outlined in FIG. 4. Data in FIG. 1 - 3 is collected at a detector in processor 10. This detector monitors the transmission of M(x,y) by examining structure and integrity of script 1. Processor 10 writes the outcome of this process into data sets N(x,y) 15 and E(x,y) 16. The difference between E(x,y) 16 and N(x,y) 15 is processed into flux 25. Digits of 1 in this flux represent error. This flux is then compiled by processor 10 into 26 and sent with 25 to the portal 6. The user then interprets this information on portal 6.

Overall, this process is completely digital. The activating, processing and detecting are all given by monitoring the transmission of script 1. Molecule 17 is identified by the manner in which its binding, recognizing or reacting to the receptor 12 alters the transmission of this script. The error generated in this process is assembled and compiled by calculating a flux 25 and change in flux 26.

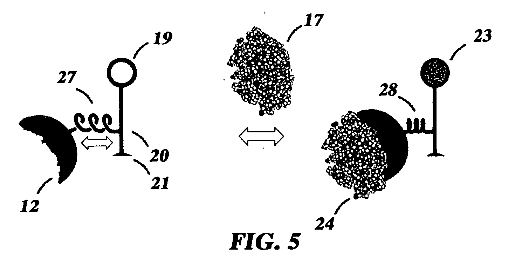

The operation of a series motors is illustrated in FIGS. 5 - 8. The embodiments given in FIG. 5 - 8 illustrate functions that underlie the regulation between interferon states 19 and 23 as shown in FIG. 4.

FIG. 5 illustrates a preferred embodiment for a spring motor. In native state 7, spring 27 is relaxed, undergoing a program of dynamic vibration. The kinetics of this spring is chosen such that interferon 19 interacts with circuit 18 at a static or calculable level. Alteration of the M(x,y) 14 at this stage is given by N(x,y) 15. Upon delivery of molecule 17, receptor 12 complexes, as in 24. This complexation constricts the vibration of the spring as shown by 28. This constriction up or down regulates the interferon's ability to alter the digital current. The transmission of M(x,y) 14 at this state is given in E(x,y) 16.

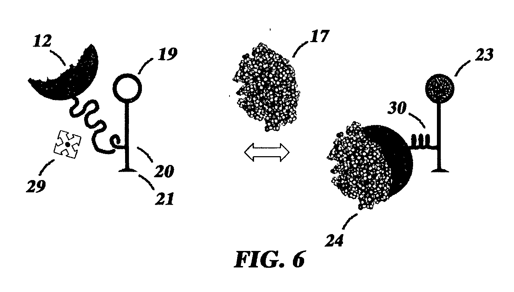

FIG. 6 illustrates a preferred embodiment for a dynamic-control motor. In native state 7, receptor 12 moves through three-dimensional space about the mounted template 20 in a regimented program of molecular dynamics. This program can be viewed as a routine of molecular animation 29. Animation 29's program is devised such that the interferon 19 has a constant or calculable or programmable interaction with circuit 18. At this stage M(x,y) 14 is transmitted to processor 10 as N(x,y) 15. As molecule 17 binds to receptor 12, the molecular dynamics of 29 becomes restricted. This converts interferon 19 to 23. Interferon 23 has either more or less interaction with circuit 18 than 19. This change respectively increases or decreases the distortion in the transmission of M(x,y) 14 through circuit 18 as given in E(x,y) 16.

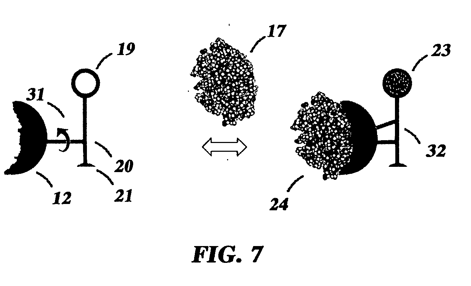

FIG. 7 illustrates a preferred embodiment for a rotary motor. Receptor 12 rotates about an axle attached to template 20. Template 20 is held stationary through mount 21. The physics of this spin are given by the nature of the axle, receptor 12, template 20 and mount 21. In native state 7, this spin 27 regulates integrator 19's interaction with circuit 18. In this state M(x,y) 14 is transmitted through circuit 18 as N(x,y) 15. The binding of molecule 17 to receptor 12 alters the motor's spin in a manner that either up or down regulates the interaction between interferon 23 and circuit 18. This change respectively increases or decreases the distortion seen in transmitting M(x,y) 14 through circuit 18 as given in E(x,y) 16.

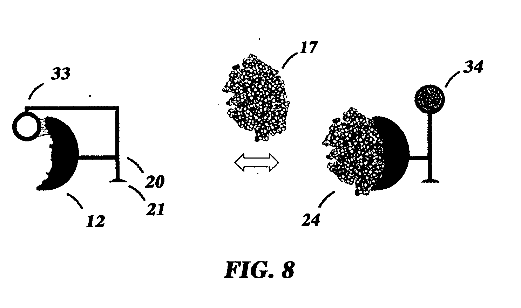

FIG. 8 illustrates a preferred embodiment for a switchable molecular motor. In native state 7, interferon 19 is restricted from accessing circuit 18 either through a covalent or non-covalent interaction. M(x,y) 14 is transmitted through circuit 18 as N(x,y) 15. As molecule 17 binds or recognizes or reacts with receptor 12, the bond or interaction is destroyed and the interferon is released as 23. Interferon 23 can now contact circuit 18. This contact either up or down regulates the distortion observed when transmitting M(x,y) 14 through circuit 18, as given in E(x,y) 16.

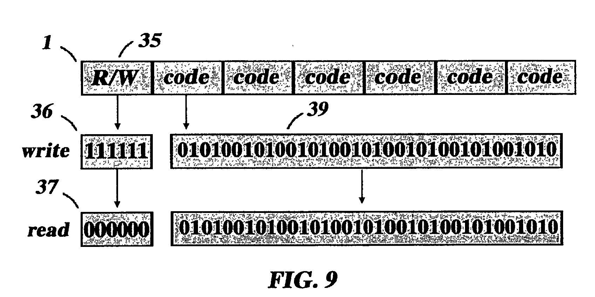

A powerful feature of this invention lies in its use of digital data in form of a single microelectronic script 1. The structure of this script is given in FIG. 9. The bit length in FIG. 9 is given for explanation purposes only. For the method to function, script 1 can be of any size as long as its size remains constant throughout the application. Script 1 begins with instructor code 35. This code determines the function and transmission of the current. As shown in FIG. 9, a six-digit code of 111111 is used to direct the code to write channel 2. This code is called write code 36. Alternatively instructor code 35 can be used to direct the script to read channel 1. This manipulation is given by changing the six-digit code from 111111 to 000000 as shown in 37. A series of other manipulations including security, communication, and delay protocols could also be included in this code. Since error is also generated in transmission of the instructor, 35 must have a high degree of redundancy. For 36 and 37, this is accommodated using six identical digits. The addition of other instructor functions would therefore require a larger bit length.

The instructor code 35 is followed by a series of redundant molecular codes 38. For this example, six 35-digit molecular codes were used. At this depth, over 34,300,000,000 molecular entities could be encoded in a single digital system.

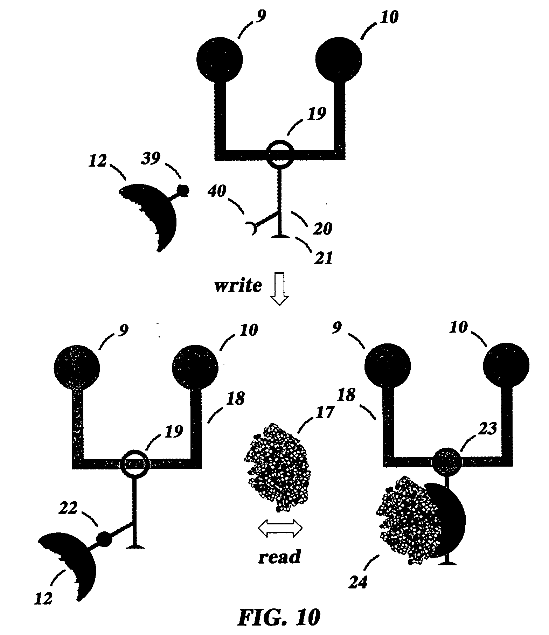

Script 1 is used command the reading, writing, display and communication of information from the mI/O. As aforementioned, the integrator's function begins writing molecular queries on the mI/O. An overview this process is provided in FIG. 10. The user starts the integrator by loading microelectronic script 1 into portal 6. This can be done through a combination of software, digital storage devices, networks, databases and/or communicators. The script begins with write code 36. This code is directed from portal 6 through write channel 3 to the mI/O 4. At mI/O 4 the code instructs a molecular printing system to deliver receptor 39 to the template of molecular motor 40. Motor 40 is held to a surface through mount 21. Reaction between the termini in 39 and 40 generate native motor 11. Once coupled, the write code 36 is changed to read code 37 and the script is returned to portal 6. The user can the apply the device to screen for molecules that bind to receptor 12.

The user exposes motor 5 to a sample containing an array of molecular species. The user then instructs portal 6 to send the microelectronic script 1 with tag 37 to mI/0 4. Tag 37 now guides the code through read channel 2. This script is then sent to mI/O 4's motor 5. The script enters through creator 9. As the script passes through motor 5, its structure is distorted by the integrator, which in turn is regulated by the binding of molecule 17 and receptor 12. The script is collected at processor 10 before and after exposure to the sample. The collected data 15 and 16 are returned to portal 6 where they is processed into 25 and 26 and delivered to the user through an audio and video display. If the sample contained molecule 17 an increased number of digits of 1 are found in flux 25. This flux can calibrated by comparison with internal and/or external controls.

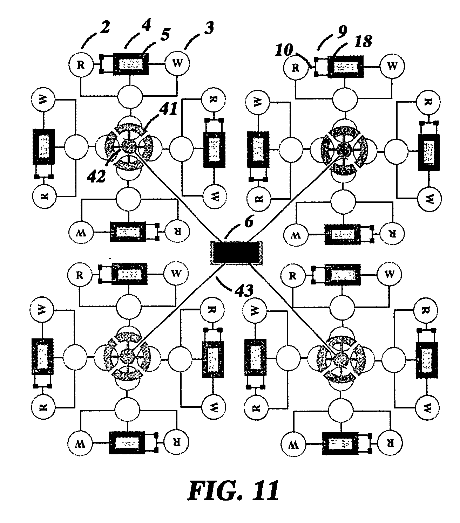

The aforementioned system can be expanded to matrixes of molecular events by networking series of integrators on a single component. One preferred embodiment for this matrix is given in FIG. 11. Here, a group of four integrators are linked through connector 41 to regional portal 42. Portals 42 can either be placed on above or below of the integrators. Portals 42 geometrically address the network. The circuit and therein expediting trouble seeking and data integration. The regional portals 42 are connected through wires 43 to the prime portal 6. Such construct is most efficiently developed on a two faced chip by dividing the mI/O 4 and the microelectronic components 2, 3, 6, 42 and 43 onto separate faces and linking them through the chip with 41. Only the integrator plane must be exposed to the molecular or biological sample, therefore minimizing unwanted contact between the molecular sample and electrical components.

This invention provides several advantages over the current approach used to screen molecules. By using a molecular motor, the signalling event transfers from direct initiation by the binding of a molecule to receptor to a signal that alters - for example allosterically - a motorized physical function (i.e., spin, rotation, dynamics or switching). This transfer reduces the systems dependence on the properties of receptor 12, molecule 17 and complex 24. The system given in this invention learns the behaviour of a receptor 12 - molecule 17 paring examining the effect of their complexation on a motor that indirectly alters M(x,y) 14 through an interferon 23. The system is directly applicable to screen for molecules where surface bound receptor systems exist. This includes resequencing of DNA using surface-matrixes of oligonucelotide probes or natural products or synthetic probes; receptor-interactions, including protein-protein, protein-carbohydrate, protein - small molecule, antibody-hapten, and synthetic molecular recognition complexes.

This invention advantageously uses digital data in form of a single digital script 1 to read, write and command the device, as given by the method of DITOM. The use of a single script profoundly simplifies the informational requirements of molecular diagnostics. Moreover, combining this script with digital microelectronic interferometry profoundly enhances the communication between molecules in mI/O 4 and microelectronic components in creator 9, portal 6 and processor 10. The data collected by this integrator can be directly read using modern telecommunication and computing instruments. Its structure can be programmed and easily be incorporated into mainstream digital formats including but not excluded to that given in UNIX or Java or Linux or DOS or MacOS, PalmOS or SMS or WAP or HTML.

The use of a single script also benefits the method's analytical control. The accuracy of this method is limited by the requirement to produce one digit of error in the flux. Optimal accuracy is given by the inverse of the bit length. Using the system in FIG. 9, one can access an accuracy within 0.48% as given by the requirement for 1 erroneous bit in a total of 270 bits. This accuracy of can by tuned by adjusting the size of script 1. This allows the user to choose regulate both the time and precision for a given assay (i.e., at transmission rate 0.1 GHz the user can screen script containing 210 digits in 2.1 microseconds). This extends a practical tool box for quality control and calibration.

Most importantly, digital script 1 named molecule 17. The integrator, as given in DITOM, operates by monitoring how 17 altered the spelling of script 1. This advantage becomes clear through analogy. The current convention in molecular diagnostics monitors how the molecule interacts with a electrical, chemical or optical field. This is analogous to monitoring the amount of ink or pixels used to display word MOM. Spelling errors such as WOW are much easier to find and comprehend than determining the differences in the amount of ink or pixels used in MOM and WOW.

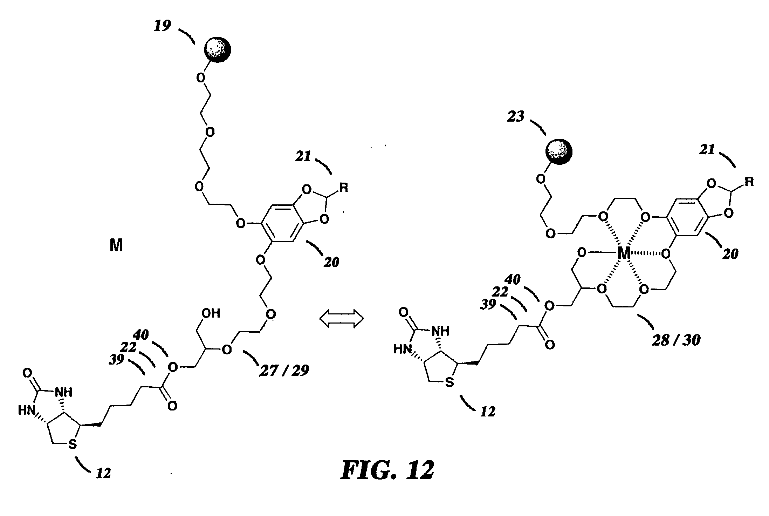

Some of the numerous possible examples of this system are provided in FIGS. 12-15. FIG. 12 provides an embodiment of a molecular motor that is regulated both by spring-action (FIG. 5) and dynamic restriction (FIG. 6). This motor responds to the protein streptavidin through the biotin moiety in its receptor 21. In native state, motor 11 shown on the left of FIG. 12 exists in matrix of conformations through rotational freedom about 28 single bonds. The dynamics of this system creates a map illustrating relative movement of receptor 12 vs. interferon 19. Upon addition of a chelate metal ion, 27/29 exists in equilibrium with closed state 28/30. This closure acts both as a dynamic constriction and as a spring. In the closed state interferon 23 is fixed as compared to 19. Due to the structural features of its binding site, streptavidin can only bind to the biotin label in the non-chelated complex. Therefore motor molecules that bind to streptavidin are locked in 27/29. This locking event alters the positioning of the interferon, which in this case increases the ability of the interferon to contact a distant electronic circuit, therein creating error in 25.

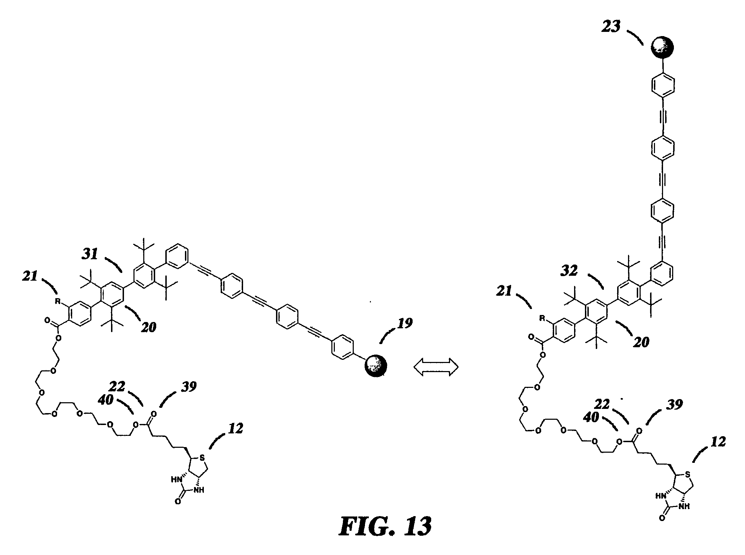

FIG. 13 provides an examples of a rotary motor. In the native state, this motor undergoes rotation around its central single bond providing a series of conformers including 31 and 32. In 31, the interferon is held in a different position with regards to 23 in the conformer 32. The binding of streptavidin to this system restricts the rotation about 32 by adjusting both the electronic character and steric congestion of the core 20. This again changes the molecular dynamics of the system therein altering the relative positioning of the interferon (i.e. 19 vs. 23). This change in positioning therein alters the ability of the interferon with the flow of current in a circuit.

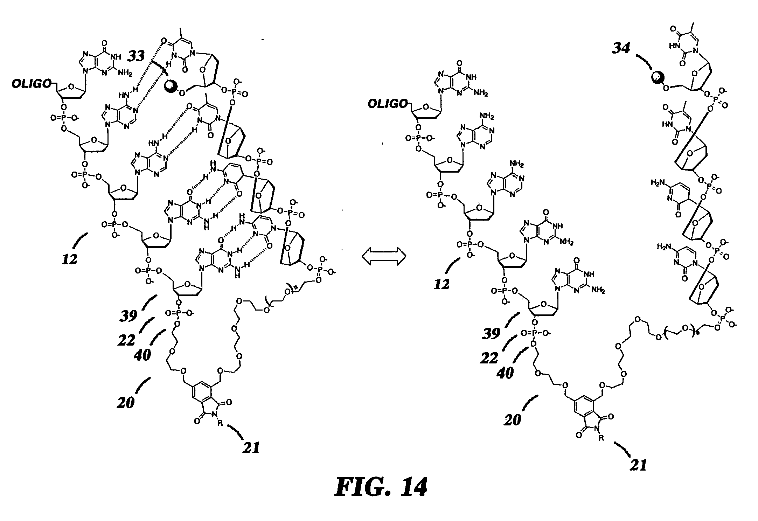

FIG. 14 provides an example of a switch motor for DNA analysis. In the native state, the motor exists in equilibrium between a bound (left of FIG. 14) and unbound state (right of FIG. 14). In bound state, a small antisense oligonucleotide intramolecularly couples with an oligonucleotide sequence. The size of the oligonucleotide sequence is abbreviated here by 5, and is typically depicted by 15 to 30 oligonucleotides. As the interaction between the small antisense sequence and host oligonucleotide is weak the system exists in equilibrium, which in part is tuned length of the antisense sequence. This equilibrium juxtapositions or switches that position of the interferon between states 33 and 34. As a sample of DNA is added that is complementary to the oligonucleotide sequence. The system is forced into the free state, therein forcing the dynamics of the interferon to state 34. This positional change serves to regulate the interferon's ability to reach a circuit, therein manipulating error in transmitting 14 as shown by 25.

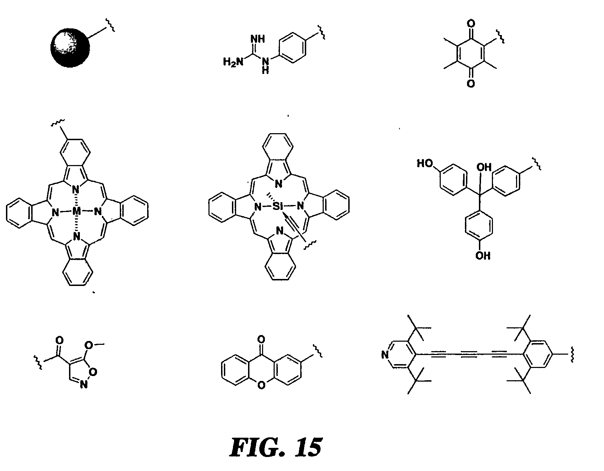

FIG. 15 provides some of the numerous examples of potential embodiments for the structure of the interferon as shown by a sphere in FIGS. 12-14. These moieties that serve as electron sinks or wells and offer a variety tools to alter the digitally encoded M(x,y) 14.

The invention presents a method and a digital electronic device to create, read, write and communicate molecular information. An apparatus that identifies molecular species by conducting a digitally-encoded microelectronic script 1 through a integrated circuit that contains a read channel 2, a write channel 3 and molecular input/output device 4 containing a molecular motor 5, and portal 6. The device uses a digital script 1 to organize, create and read an array of specific molecular queries. Its system can be networked to provide microelectronic affinity matrixes that directly communicate with instruments of telecommunication and computing.

It will be apparent to those skilled in the art that, the disclosed invention may be modified in numerous ways and may assume many embodiments other than the preferred form used herein to illustrate the method as describe above. Accordingly, it is intended that the appended claims cover all modifications of the invention which fall within the true spirit and scope of the invention.

## Claims

1. Method for reading, writing and communicating information on molecular species, the method comprising
generating or transiting digital data (1) by or via a user-accessible portal (6), transiting the digital data (1) via at least one read channel (2) to at least one microelectronic molecular input/output device (4),
transiting the digital data (1) through the at least one molecular input/output device (4),
transiting the digital data via at least one write channel (3) to the portal (6).

2. Method according to claim 1, wherein
the at least one microelectronic molecular input/output device (4) comprises a creator (9), a digital processor (10) and a molecular motor (5).

3. Method according to any of claims 1 or 2, wherein
the portal (6) is operated by an entry device, preferably comprising a keyboard, a mouse and/or a number pad, and/or through a communication unit, preferably comprising a telephone, a cellular phone, a modem, an infrared file exchange and/or a pulsed-laser.

4. Method according to any of the preceding claims, wherein
the digital data (1) are generated or transited as pulses of electronic digital current, preferably in a waveform, more preferably with box wave character.

5. Method according to claim 4, wherein,
the pulses of generated or transited electronic digital current have a rate that exceeds about 0,1 GHz.

6. Method according to any of the preceding claims, wherein
the digital data (1) are amended by the dynamics of the molecular motor (5).

7. Method according to claim 6, wherein
the dynamics of the molecular motor (5) is amended by a binding, recognizing or reacting of at least one molecule (17) with a receptor (12) comprised in the molecular motor (5).

8. Method according to claim 7, wherein
the digital data (15) received at the processor (10) without a molecule binding, recognizing or reacting to the receptor (12) and the digital data (16) received at the processor (10) with at least one molecule (17) binding, recognizing or reacting to the receptor (12) are compared.

9. Method according to claim 8, wherein
the identity of at least one molecule (17) is specified by comparing the digital data (15) received at the processor (10) without a molecule binding, recognizing or reacting to the receptor (12) and the digital data (16) received at the processor (10) with at least one molecule binding, recognizing or reacting to the receptor (12) with regards to the structure of the digital data received at the molecular input/output device (4).

10. Apparatus for reading, writing and communicating information on molecular species comprising an integrated circuit containing
a user-accessible portal (6);
means for generating or transiting digital data (1) by or via the portal (6);
at least one read channel (2);
means for transiting the digital data (1) through the read channel (2);
at least one microelectronic molecular input/output device (4);
means for transiting the digital data (1) from the read channel (2) to the at least one microelectronic molecular input/output device (4);
at least one write channel (3);
means for transiting the digital data (1) from the molecular input/output device (4) via the write channel (3) to the portal (6).

11. Apparatus according to claim 10, the at least one microelectronic molecular input/output device (4) comprising
a creator (9), a digital processor (10), and a molecular motor (5).

12. Apparatus according to any of claims 10 to 12, wherein
in the microelectronic molecular input/output device (4) the creator (9) and/or processor (10) are a transistor or network of transistors.

13. Apparatus according to claim 11 or 12, wherein
the creator (9 ) and processor (10) are displayed within an electronic circuit.

14. Apparatus according to any of claims 10 to 13, further comprising
means for generating or transiting digital data (1) as pulses of electronic digital current, preferably in waveform, more preferably with box wave character.

15. Apparatus according to claim 14, comprising
means for generating electronic digital current at a rate that exceeds about 0,1 GHz.

16. Apparatus according to any of claims 11 to 15, wherein
the creator (9) obtains its digital data from the portal (6) through a bioinformatic database.

17. Apparatus according to any of claims 11 to 16, wherein
the motor (5) comprises a template (20), a mount (21), a linker (22) to attach a molecular receptor, a molecular receptor (12) and an interferon (19; 23).

18. Apparatus according to claim 17, comprising means for encoding at least one molecule by binding, recognizing or reacting to the receptor (12) in the molecular motor (5).

19. Apparatus according to any of claims 17 to 18, further comprising means for comparing the digital data (15) received at the processor (10) without a molecule binding, recognizing or reacting to the receptor (12) and the digital data (16) received at the processor (10) with at least one molecule (17) binding, recognizing or reacting to the receptor (12).

20. Apparatus according to any of claims 17 to 19, further comprising means for identitifing at least one molecule (17) by comparing the digital data (15) received at the processor (10) without a molecule binding, recognizing or reacting to the receptor (12) and the digital data (16) received at the processor (10) with at least one molecule binding, recognizing or reacting to the receptor (12) with regards to the structure of the digital data received at the molecular input/output device (4).

21. Apparatus according to any of claims 11 to 20, wherein
the molecular motor (5) comprises a molecular spring, a molecular rotor or a molecular switch.

22. Apparatus according to any of claims 19 to 21, wherein
the linker (22) contains a functionality that attaches to at least one molecule of a oligonucleotide, a protein, a carbohydrate, a carboxylic acid or ester.

23. Apparatus according to any of claims 19 to 22, wherein
the mount (21) contains functionality that allows it to be attached to polycarbonate, glass, or gold.

24. Apparatus according to any of claims 19 to 23, wherein
the interferon (19; 23) changes the electrical properties of a microelectronic channel upon binding or recognizing or reacting of at least one molecule to the molecular motor's receptor (12).

25. Apparatus according to any of claims 10 to 24, wherein
the information management of the motor (5) is based on the operating system of DITOM.

26. Apparatus according to any of claims 10 to 25, further comprising
an entry device, preferably a keyboard, a mouse and/or a number pad, and/or a communication unit, preferably comprising a telephone, a cellular phone, a modem, an infrared file exchange and/or a pulsed-laser to operate the portal (6).
